# EUROPÄISCHE PATENTSCHRIFT

(11) **EP 2 027 885 B1**
(45) Veröffentlichungstag und Bekanntmachung des Hinweises auf die Patenterteilung: **29.12.2010**
(21) Anmeldenummer: 08012897.8
(22) Anmeldetag: 17.07.2008
(51) Int. Cl.: A61M 39/24, F16K 15/14

(54) **Rückschlagventil, insbesondere für medizinische Anwendungen**
Non-return valve, in particular for medical applications
Clapet anti-retour, en particulier pour des applications médicales

(30) Priorität: 23.08.2007 DE 202007011811 U; 11.09.2007 DE 202007012681 U
(43) Veröffentlichungstag der Anmeldung: 25.02.2009
(73) Patentinhaber: Filtertek B.V., County of Limerick (IE)
(72) Erfinder: Costello, Kieran, Cullenagh Ballina/Killaloe Co. Clare (IE)
(74) Vertreter: Beck, Alexander

(56) Entgegenhaltungen:
- EP-A- 0 247 824
- EP-A- 1 099 455
- WO-A-96/24791
- FR-A- 1 410 619

## Beschreibung

Die Erfindung betrifft ein Rückschlagventil, insbesondere für medizinische Anwendungen, mit einem ersten Schlauchanschlussgehäuse und einem zweiten Schlauchanschlussgehäuse und einer zwischen beiden Schlauchanschlussgehäusen angeordneten Membranscheibe aus flexiblem Werkstoff, welche bei Überdruck in einem Eingangsdurchlass im ersten Schlauchanschlussgehäuse von einem ringförmigen, einen mit dem Eingangsdurchlass verbundenen Einlassraum umgebenden Ventilsitz abhebbar ist und die bei Überdruck in einem Ausgangsdurchlass sicher und in Minimalzeiten auf den Ventilsitz andrückbar ist, wobei die Membranscheibe an ihrem äußeren Umfangsbereich mit einem Ringwulst versehen ist, welcher in einander gegenüberliegenden Ringnuten der Schlauchanschlussgehäuse aufgenommen ist, wobei die Membranscheibe radial außerhalb des Ventilsitzes mit Öffnungen versehen ist, die mit einem Auslassraum verbunden sind.

Ein derartiges Rückschlagventil ist nach einem früheren Vorschlag der Anmelderin aus dem Europäischen Patent 0 612 537 bekannt.

Derartige Rückschlagventile werden in der Medizintechnik für die Leitung von Infusionssystemen, Spritzen, Diagnoseausrüstungen, intravenösen Schlauchleitungen und in Verbindung mit Spritzpumpen verwendet. Bei Schließzeiten von wenigen Bruchteilen von Sekunden müssen derartige Ventile sicher schließen, um jeden Rückfluss möglicherweise kontaminierter Flüssigkeiten zu vermeiden. Aufgrund der Tatsache, dass derartige Rückschlagventile in der Medizintechnik ausschließlich als Einwegartikel verwendet werden, ist es gleichzeitig unverzichtbar, dass die Herstellung einerseits extrem wirtschaftlich und andererseits statistisch sehr genau ist. Ferner sind sehr strenge gesetzliche Vorschriften und Untersuchungen vorgesehen, um eine gleich bleibende einheitliche Funktionssicherheit sicherzustellen. Diese Voraussetzungen werden beispielsweise in Deutschland durch den TÜV genau geprüft, ehe bei positiven Ergebnissen der Prüfung die Freigabe für medizinische Anwendung erfolgt. Zwar werden bei dem oben erwähnten Rückschlagventil nach dem früheren Vorschlag der Anmelderin diese Anforderungen durchaus erfüllt, indem bei dieser bekannten Konstruktion die den Ringwulst am Umfangsbereich der Membran aufnehmenden Ringnuten derart gestaltet sind, dass bei der Montage der beiden Schlauchanschlussgehäuse radial gerichtete Zugkräfte auf die Membranscheibe aufgebracht werden, so dass hierdurch eine Einstellung der Zugspannung der Membran erfolgen kann, die das Anliegen an dem Ventilsitz gewährleistet und für ein ausgesprochen schnelles und sicheres Ansprechen des Rückschlagventils sorgt.

Beim praktischen Einsatz dieses bekannten Ventils hat sich jedoch gezeigt, dass der ringförmige Ventilsitz, welcher mit einer scharfen Oberkante ausgebildet ist, in vielen Fällen bei der Herstellung beschädigt werden kann, so dass die angestrebte Linienberührung an der Membranscheibe nicht immer gewährleistet ist und daher ein nicht unerheblicher Anteil an Ausschuss bei der Produktion auftreten kann. Ferner hat es sich gezeigt, dass die herzustellende Vorspannung durch Gestaltung der Ringnuten bei der Membran in manchen Fällen nicht immer sicher in statistischem Maße eingehalten werden kann.

Der Erfindung liegt daher die Aufgabe zugrunde, Ventile der eingangs definierten Art dahingehend zu verbessern, dass diese Nachteile vermieden oder zumindest gemildert werden.

Bei einem Rückschlagventil der eingangs genannten Art wird diese Aufgabe durch den kennzeichnenden Teil des Anspruchs 1 gelöst.

Es ist offensichtlich, dass durch Vermeidung des scharfkantigen ringförmigen Ventilsitzes, welcher durch die ebene ringförmige Fläche ersetzt wird, die Gefahr von Beschädigungen des Ventilsitzes während der Produktion erheblich herabgesetzt wird. Gleichzeitig bildet der an der Membranscheibe vorgesehene kreisförmige Vorsprung ein definiertes Schließglied des Ventils, durch welches aufgrund der erzielten, vergleichsweise großen Dichtfläche ein sicheres Schließen gewährleistet ist.

Indem die Höhe des Vorsprungs größer oder gleich der Wandstärke der Membranscheibe indem die Öffnungen aufweisenden Bereich zwischen dem Ringwulst und dem Vorsprung ist, wird die notwendige Vorspannung der Membranscheibe in exakt definierter Weise durch die Höhe des Vorsprungs erzielt, so dass die Unsicherheiten, die sich aus der Erzeugung der Vorspannung mittels radialer Zugspannung ergeben können, vermieden wird.

Im Einzelnen ist es insbesondere bevorzugt, auf der Oberseite und der Unterseite der Membranscheibe identische Vorsprünge vorzusehen. Hierdurch wird die Herstellung des erfindungsgemäßen Rückschlagventils in automatisierter Weise erheblich erleichtert, da beim automatisierten Zusammenbau nicht auf eine besondere Orientierung der Membranscheibe geachtet werden muss.

Eine besonders bevorzugte Ausführungsform kann dadurch geschaffen werden, dass der Vorsprung in einem zentralen kreisförmigen Bereich eine Vertiefung aufweist. Hierdurch wird der Vorsprung auf die ringförmige Dichtfläche reduziert und somit eine Material- und Gewichtsersparnis für die Membranscheibe erzielt.

Bevorzugt ist es, dass die Wandstärke des Bodens der Vertiefung etwa der Wandstärke der Membranscheibe mit dem die Öffnungen erhaltenden Bereich entspricht.

Im Einzelnen kann die Erfindung dadurch weitergebildet werden, dass die kreisförmige Oberkante des Vorsprungs eine Abschrägung oder Abrundung aufweist.

Ferner ist es bevorzugt, dass die ringförmige Seitenwand des Vorsprungs schräg nach außen geneigt ausgebildet ist.

Im Folgenden wird die Erfindung an Hand von in den Zeichnungen beispielhaft veranschaulichten Ausführungsformen näher erläutert. Es zeigt:
**FIGUR 1** eine schematische Schnittansicht des Rückschlagventils nach der Erfindung mit einer ersten Ausführungsform der Membranscheibe im geöffneten Zustand;
**FIGUR 2** eine stark vergrößerte Schnittansicht des Rückschlagventils gemäß Figur 1 im Bereich der Membranscheibe;
**FIGUR 3** eine Schnittansicht des Rückschlagventils gemäß Figuren 1 und 2 im geschlossenen Zustand;
**FIGUR 4** eine Figur 2 entsprechende Schnittansicht des Rückschlagventils gemäß Figur 3;
**FIGUR 5** eine stark vergrößerte perspektivische Ansicht der Membranscheibe in dem Ventil gemäß Figur 1;
**FIGUR 6** eine Draufsicht auf die Membranscheibe gemäß Figur 2;
**FIGUR 7** eine perspektivische Schnittansicht der Membranscheibe gemäß den **Figuren 2** und 3;
**FIGUR 8** eine Schnittansicht der Membranscheibe gemäß Figuren 2 bis 4;
**FIGUR 9** eine Figur 2 entsprechende perspektivische Ansicht einer zweiten Ausführungsform der Membranscheibe;
**FIGUR 10** eine Draufsicht der Membranscheibe gemäß Figur 6;
**FIGUR 11** eine perspektivische Schnittansicht der Membranscheibe gemäß Figuren 6 und 7, und
**FIGUR 12** eine Schnittansicht der Membranscheibe gemäß den Figuren 6 bis 8.

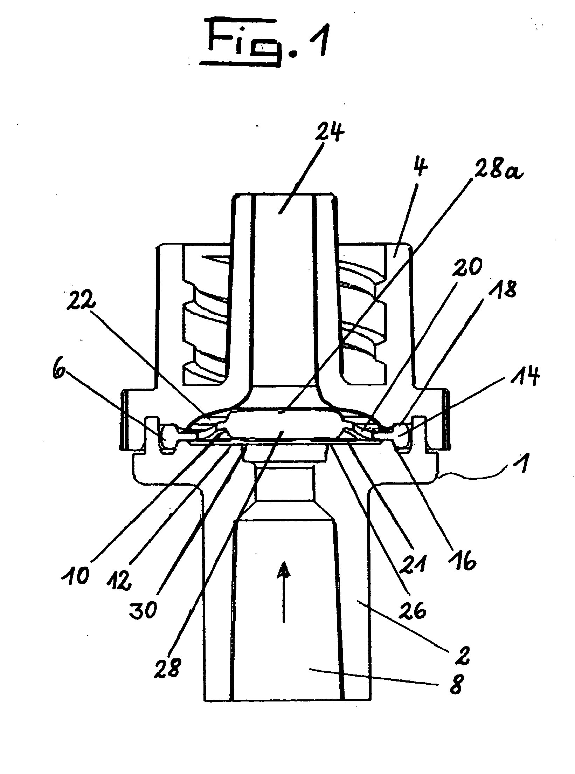

Das in den Figuren 1 bis 4 gezeigte Rückschlagventil 1 besteht aus einem ersten Schlauchanschlussgehäuse 2 und einem zweiten Schlauchanschlussgehäuse 4, welche beispielsweise durch Spritzgießen aus Kunststoff hergestellt sind, sowie einer zwischen den beiden Schlauchanschlussgehäusen 2 und 4 eingespannten Membranscheibe 6. Die Membranscheibe 6 ist aus einem flexiblen Werkstoff, beispielsweise Silikon, hergestellt.

Das erste Schlauchanschlussgehäuse 2 ist mit einem Eingangsdurchlass 8 versehen, welcher in einem Einlassraum 10 mündet. Der Einlassraum 10 ist von einem ringförmigen Ventilsitz 12 umgeben, an welchem die Membran 6 in der weiter unten erläuterten Weise anliegt.

Die Membran 6 ist an ihrem äußeren Umfangsbereich mit einem Ringwulst 14 versehen, welcher dort beispielsweise an die Membran 6 angespritzt ist, falls diese ebenfalls durch Spritzgießen des Silikons hergestellt wurde. In dem ersten Schlauchanschlussgehäuse 2 ist in dessen Stirnfläche eine Ringnut 16 ausgebildet, wobei eine Ringnut 18 des zweiten Schlauchanschlussgehäuses 4 dieser im zusammengebauten Zustand gegenüberliegt. Beim Zusammenbau der beiden Schlauchanschlussgehäuse 2 und 4 wird die Ringwulst 14 in die einander gegenüberliegenden Ringnuten 16 und 18 der beiden Schlauchanschlussgehäuse 2 und 4 aufgenommen und die Membranscheibe 6 gleichzeitig gespannt.

Wie ferner aus den Figuren 1 bis 4 ersichtlich, ist die Membranscheibe 6 radial außerhalb des Ventilsitzes 12 mit Öffnungen 20 versehen, welche auf einem Radius angeordnet sind und welche einen radial außerhalb des Ventilsitzes 12 liegenden Ringraum 21 im ersten Schlauchanschlussgehäuse 2 mit einem Auslassraum 22 des zweiten Schlauchanschlussgehäuses 4 verbinden, der seinerseits mit dem Ausgangsdurchlass 24 des zweiten Schlauchanschlussgehäuses 4 in Verbindung steht.

Wie aus den Figuren 1 bis 4 ersichtlich, ist der Ventilsitz 12 als ebene ringförmige Fläche 26 ausgebildet, welche den Einlassraum 10 auf der der Membranscheibe 6 zugewandten Seite umgibt.

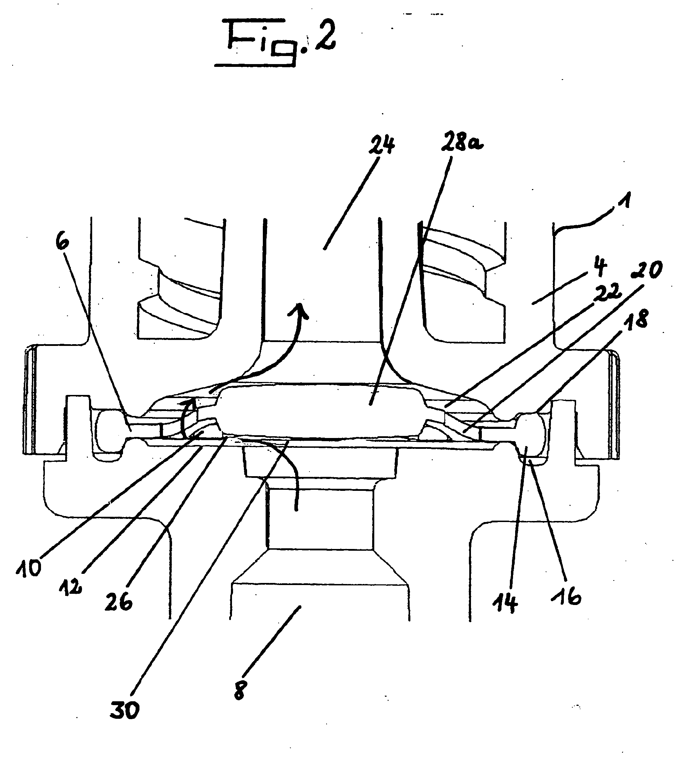

Die Figuren 1 und 2 zeigen die erste Ausführungsform des Rückschlagventils 1 im geöffneten Zustand, bei welchem der weiter unten näher erläuterte kreisförmig ausgebildete Vorsprung 28 der Membranscheibe 6 von dem Ventilsitz 12, welcher durch die ebene ringförmige Fläche 26 gebildet ist, abgehoben ist. Somit kann das Medium von dem Eingangsdurchlass 8 in den Einlassraum 10 strömen und gelangt durch die Öffnungen 20 in den Auslassraum 22, von welchem das Medium dann zu dem Ausgangsdurchlass 24 strömen kann.

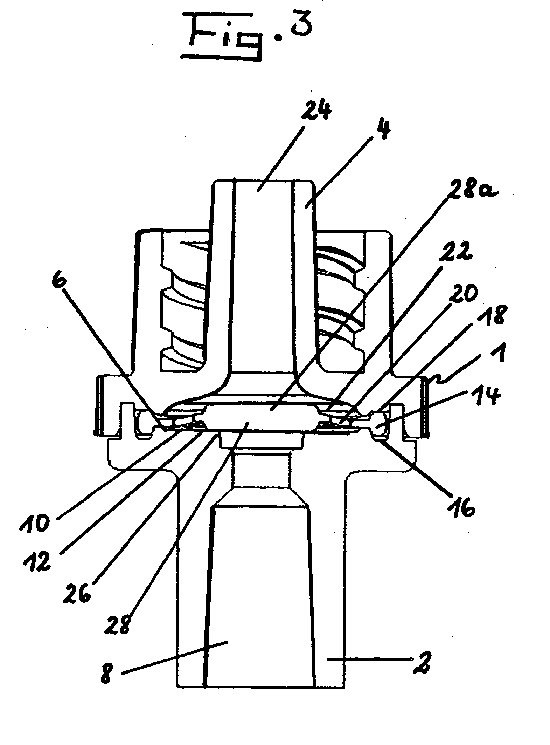

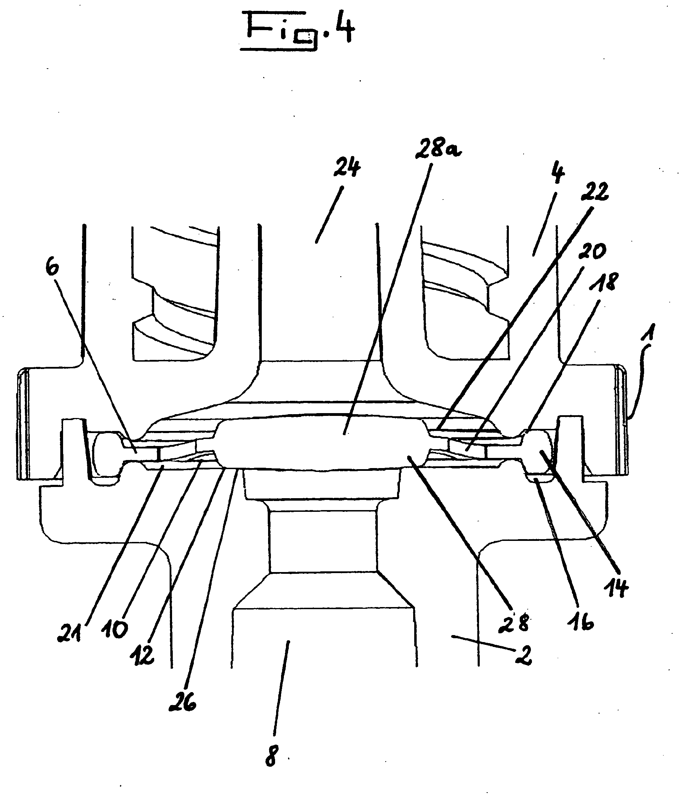

Bei dem in den Figuren 3 und 4 veranschaulichten geschlossenen Zustand des Ventils sitzt der Vorsprung 28 auf der den Ventilsitz 12 bildenden ringförmigen ebenen Fläche 26 auf, so dass der Eingangsdurchlass 8 von dem Einlassraum 10 durch die Membranscheibe 6 getrennt ist und kein Medium vom Ausgangsdurchlass 24 zurück zum Eingangsdurchlass 8 strömen kann.

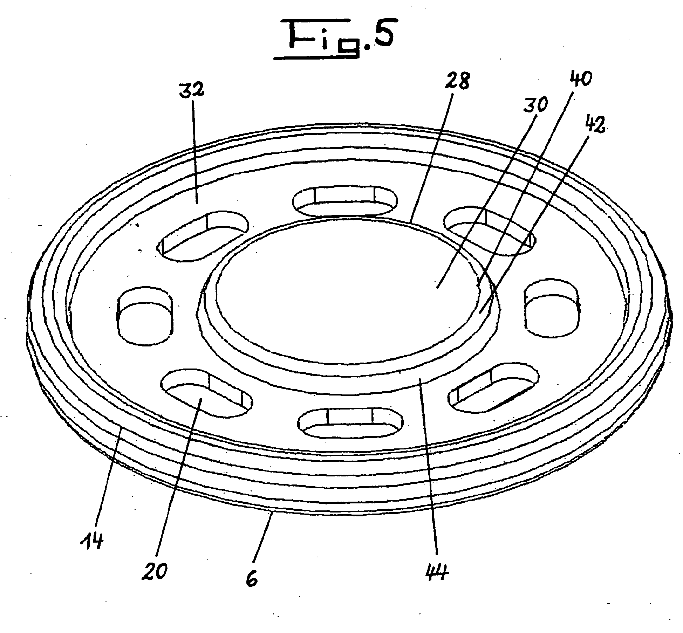

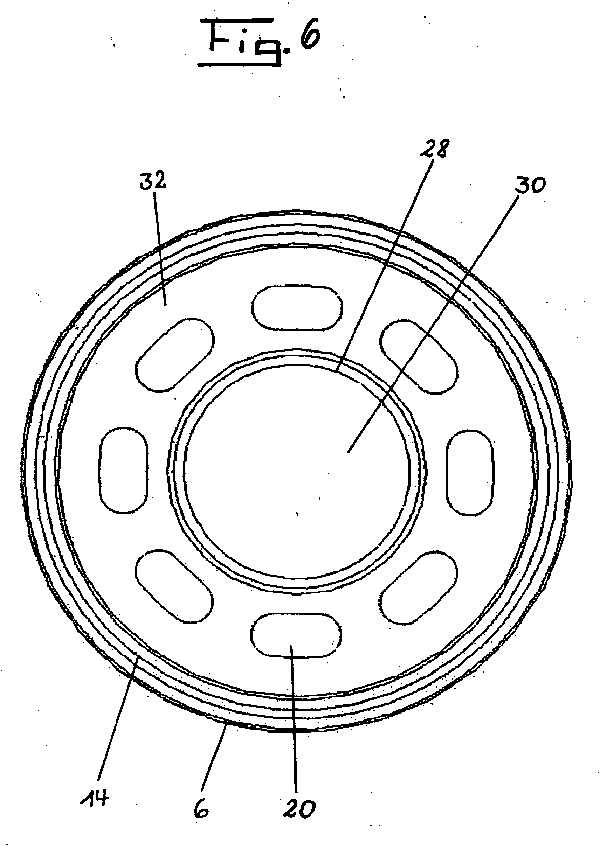

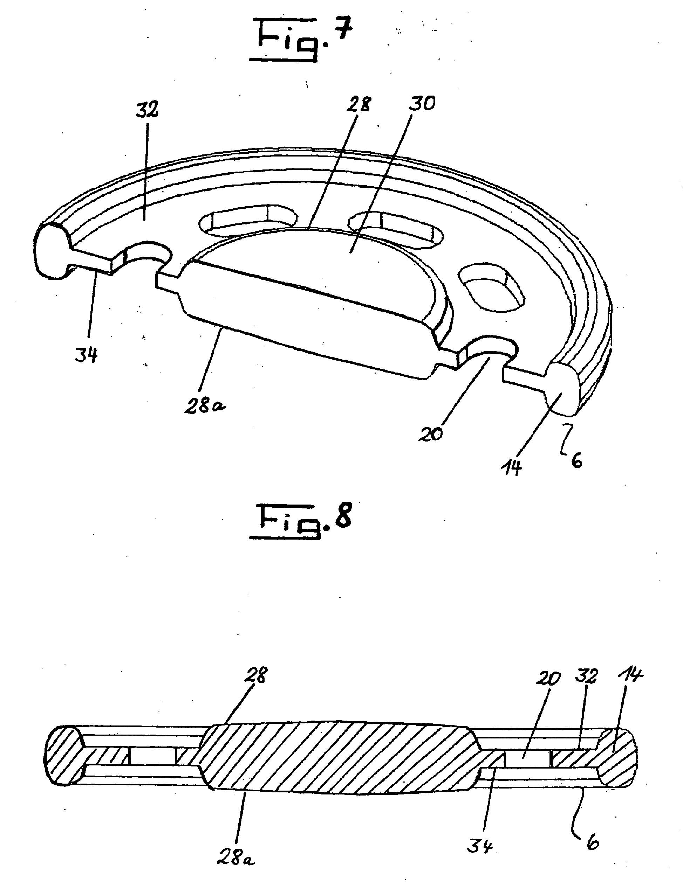

Wie ferner in einer ersten Ausführungsform, insbesondere aus den Figuren 5 bis 8, ersichtlich, ist die Membranscheibe 6 radial innerhalb der Öffnungen 20 mit einem einstückigen, kreisförmig ausgebildeten Vorsprung 28 versehen. Der Vorsprung 28 weist zumindest in dem dem Ventilsitz 12 gegenüberliegenden Bereich eine ebene Oberfläche 30 auf, welche dem Ventilsitz 12 zugewandt ist.

Der Vorsprung 28 weist eine Höhe auf, die etwa größer oder gleich der Wandstärke der Membranscheibe 6 in dem Bereich zwischen dem Ringwulst 14 und dem Vorsprung 28 ist, welcher die Öffnungen 20 enthält.

Insbesondere bevorzugt ist es, auf der Oberseite 32 und der Unterseite 34 der Membranscheibe 6 identische Vorsprünge 28 und 28a vorzusehen. Hierdurch wird vermieden, dass beim automatisierten Zusammenbau des Rückschlagventils 1 gemäß den Figuren 1 bis 4 auf eine spezielle Orientierung der Membranscheibe 6 geachtet werden muss.

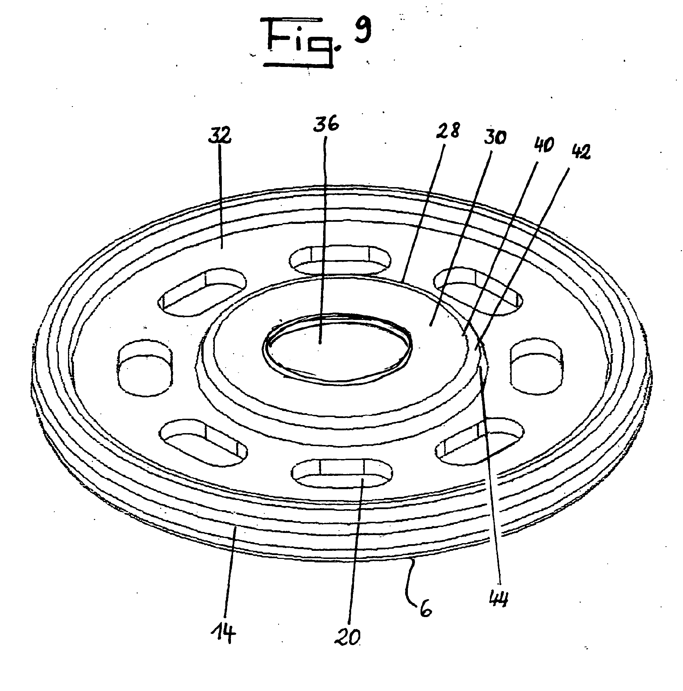

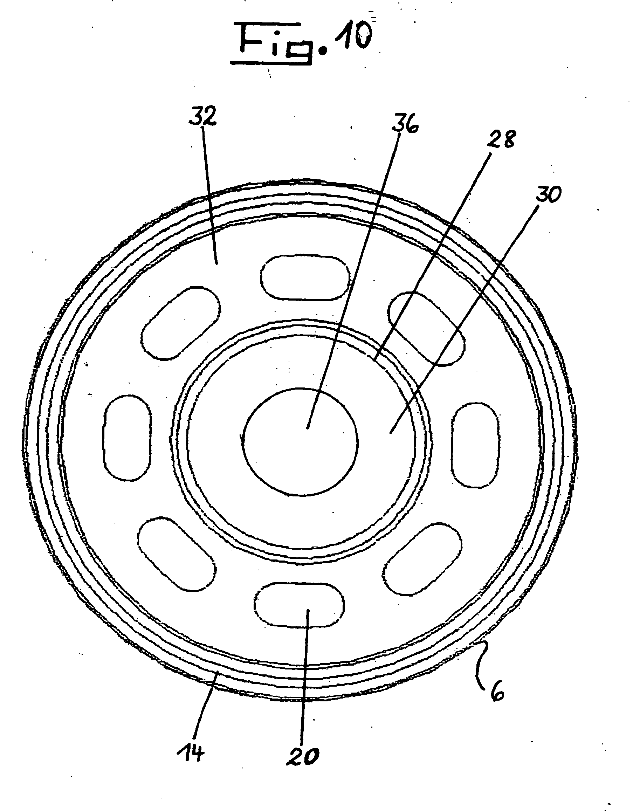

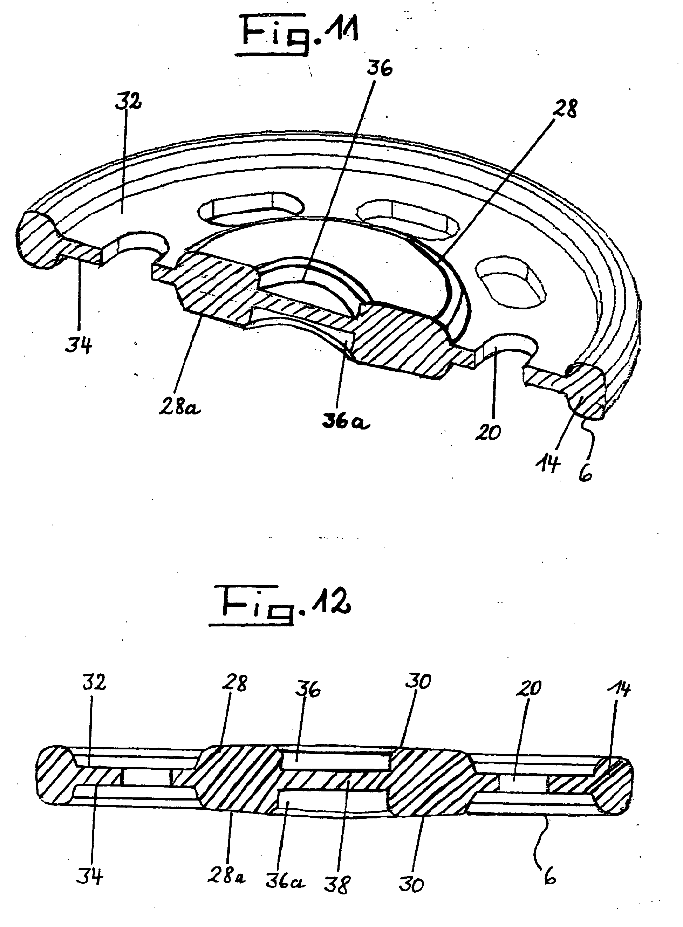

Bei der in den Figuren 9 bis 12 dargestellten Ausführungsform, welche in wesentlichen Einzelheiten der Ausführungsform gemäß den Figuren 5 bis 8 entspricht, ist in einem zentralen kreisförmigen Bereich des Vorsprungs 28 auf der Oberseite 32 der Membranscheibe 6 und des Vorsprungs 28a auf der Unterseite 34 der Membranscheibe 6 eine Vertiefung 36 ausgebildet, welche ebenfalls kreisförmig gestaltet ist. Die Wandstärke des Bodens 38, welche zwischen den Vertiefung 36 auf der Oberseite 32 des Vorsprungs 28 und der Vertiefung 36 auf der Unterseite 34 der Membranscheibe 6 im Vorsprung 28a verbleibt, entspricht etwa der Wandstärke der Membranscheibe 6 in dem die Öffnungen enthaltenden Bereich.

Wie ferner bei beiden Ausführungsformen gezeigt, weist die kreisförmige Oberkante 40 des Vorsprungs 28 und des Vorsprungs 28a eine Abschrägung 42 auf, die auch als Abrundung ausgebildet sein kann. Ferner ist die ringförmige Seitenwand 44 der Vorsprünge 28, 28a schräg nach außen geneigt ausgebildet. Beide Merkmale können auch bei der Kante bzw. Seitenwand der Vertiefungen 36, 36a vorgesehen sein.

Sämtliche aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

### BEZUGSZEICHENLISTE

- 1 =: Rückschlagventil
- 2 =: erstes Schlauchanschlussgehäuse
- 4 =: zweites Schlauchanschlussgehäuse
- 6 =: Membranscheibe
- 8 =: Eingangsdurchlass
- 10 =: Einlassraum
- 12 =: Ventilsitz
- 14 =: Ringwulst
- 16 =: Ringnut
- 18 =: Ringnut
- 20 =: Öffnungen
- 21 =: Ringraum
- 22 =: Auslassraum
- 24 =: Ausgangsdurchlass
- 26 =: ringförmige Fläche
- 28,28a: = Vorsprung
- 30 =: Oberfläche
- 32 =: Oberseite v. 6
- 34 =: Unterseite v. 6
- 36,36a: = Vertiefung
- 38 =: Boden v. 36
- 40 =: Oberkante v. 28
- 42 =: Abschrägung
- 44 =: Seitenwand v. 28

## Patentansprüche

1. Rückschlagventil (1), insbesondere für medizinische Anwendungen, mit einem ersten Schlauchanschlussgehäuse (2) und einem zweiten Schlauchanschlussgehäuse (4) und einer zwischen den beiden Schlauchanschlussgehäusen (2, 4) angeordneten Membranscheibe (6) aus flexiblem Werkstoff, welche bei Überdruck in einem Eingangsdurchlass (8) in einem ersten Schlauchanschlussgehäuse (2) von einem ringförmigen, einen mit dem Eingangsdurchlass (8) verbundenen Einlassraum (10) umgebenden Ventilsitz (12) abhebbar ist und die bei Überdruck in einem Ausgangsdurchlass (24) sicher und in Minimalzeiten auf den Ventilsitz (12) andrückbar ist, wobei die Membranscheibe (6) an ihrem äußeren Umfangsbereich mit einem Ringwulst (14) versehen ist, welcher in einander gegenüberliegenden Ringnuten (16, 18) der Schlauchanschlussgehäuse (2, 4) aufgenommen ist, wobei die Membranscheibe (6) durch die Ringnuten (16, 18) mittels des Ringwulstes (14) gegen den Ventilsitz (12) vorgespannt ist und wobei die Membranscheibe (6) radial außerhalb des Ventilsitzes (12) mit Öffnungen (20) versehen ist, die mit einem Auslassraum (22) verbunden sind, und wobei ferner der Ventilsitz (12) als den Einlassraum (10) auf der der Membranscheibe (6) zugewandten Seite umgebende ebene, ringförmige Fläche (26) ausgebildet ist, **dadurch gekennzeichnet, dass** die Membranscheibe (6) radial innerhalb der Öffnungen (20) mit einem einstückigen, kreisförmigen Vorsprung (28, 28a) versehen ist, wobei der Vorsprung (28, 28a) zumindest in dem dem Ventilsitz (12) gegenüberliegenden Bereich eine dem Ventilsitz (12) zugewandte ebene Oberfläche (30) aufweist, welche mit dem Ventilsitz (12) in einem breiten, ringförmigen Bereich in Eingriff gelangt, dass auf der Oberseite (32) und Unterseite (34) der Membranscheibe (6) identische Vorsprünge (28, 28a) vorgesehen sind, und dass die Höhe des Vorsprungs (28, 28a) größer oder gleich der Wandstärke der Membranscheibe (6) in dem die Öffnungen (20) aufweisenden Bereich zwischen Ringwulst (14) und Vorsprung (28) ist.

2. Rückschlagventil nach Anspruch 1, **dadurch gekennzeichnet, dass** der Vorsprung (28, 28a) in einem zentralen kreisförmigen Bereich eine Vertiefung (36) aufweist.

3. Rückschlagventil nach Anspruch 2, **dadurch gekennzeichnet, dass** die Wandstärke des Bodens (38) der Vertiefung (36) etwa der Wandstärke der Membranscheibe (6) in dem die Öffnungen (20) enthaltenden Bereich entspricht.

4. Rückschlagventil nach einem der vorstehenden Ansprüche, **dadurch gekennzeichnet, dass** die kreisförmige Oberkante (40) des Vorsprungs (28, 28a) eine Abschrägung (42) oder Abrundung aufweist.

5. Rückschlagventil nach einem der vorstehenden Ansprüche, **dadurch gekennzeichnet, dass** die ringförmige Seitenwand (44) des Vorsprungs (28, 28a) schräg nach außen geneigt ausgebildet ist.

## Claims

1. Non-return valve (1), in particular for medical applications, with a first tube connection housing (2) and a second tube connection housing (4) and, arranged between the two tube connection housings (2, 4), a diaphragm disc (6) which is made of flexible material and which, when there is an overpressure in an inlet channel (8) in a first tube connection housing (2), can be lifted from an annular valve seat (12) surrounding an inlet chamber (10) connected to the inlet channel (8), and which, when there is an overpressure in an outlet channel (24), can be pressed reliably and within minimal times onto the valve seat (12), the diaphragm disc (6), at its outer peripheral area, being provided with an annular bead (14) which is received in mutually opposite annular grooves (16, 18) of the tube connection housings (2, 4), the diaphragm disc (6) being pretensioned against the valve seat (12) by the annular grooves (16, 18) by means of the annular bead (14), and the diaphragm disc (6) being provided, radially outside the valve seat (12), with openings (20) connected to an outlet chamber (22), and furthermore the valve seat (12) being designed as a flat annular surface (26) surrounding the inlet chamber (10) on the side directed towards the diaphragm disc (6), **characterized in that** the diaphragm disc (6) is provided, radially inside the openings (20), with a one-piece circular projection (28, 28a), the projection (28, 28a), at least in the area lying opposite the valve seat (12), having a flat surface (30) which faces the valve seat (12) and which engages with the valve seat (12) in a wide, annular area, **in that** identical projections (28, 28a) are provided on the top (32) and underside (34) of the diaphragm disc (6), and **in that** the height of the projection (28, 28a) is greater than or equal to the wall thickness of the diaphragm disc (6) in the area having the openings (20) between annular bead (14) and projection (28).

2. Non-return valve according to Claim 1, **characterized in that** the projection (28, 28a) has a depression (36) in a central circular area.

3. Non-return valve according to Claim 2, **characterized in that** the wall thickness of the bottom (38) of the depression (36) corresponds approximately to the wall thickness of the diaphragm disc (6) in the area containing the openings (20).

4. Non-return valve according to one of the preceding claims, **characterized in that** the circular upper edge (40) of the projection (28, 28a) has a bevel (42) or is rounded.

5. Non-return valve according to one of the preceding claims, **characterized in that** the annular side wall (44) of the projection (28, 28a) is designed sloping obliquely outwards.

## Revendications

1. Soupape anti-retour (1), en particulier pour des applications médicales, comprenant un premier boîtier de raccord de flexible (2) et un deuxième boîtier de raccord de flexible (4) et un disque de membrane (6) en matériau flexible, disposé entre les deux boîtiers de raccord de flexible (2, 4), qui, dans le cas d'une surpression dans un passage d'entrée (8) dans un premier boîtier de raccord de flexible (2), peut être soulevé d'un siège de soupape annulaire (12) entourant un espace d'entrée (10) connecté au passage d'entrée (8), et qui, dans le cas d'une surpression dans un passage de sortie (24), peut être pressé de manière sûre et en des temps minimes sur le siège de soupape (12), le disque de membrane (6) étant pourvu au niveau de sa région périphérique extérieure d'un bourrelet annulaire (14) qui est reçu dans des rainures annulaires opposées (16, 18) des boîtiers de raccord de flexible (2, 4), le disque de membrane (6) étant précontraint par les rainures annulaires (16, 18) au moyen du bourrelet annulaire (14) contre le siège de soupape (12) et le disque de membrane (6) étant pourvu radialement à l'extérieur du siège de soupape (12) d'ouvertures (20), qui sont connectées à un espace d'écoulement (22), le siège de soupape (12) étant réalisé en outre en tant que surface plane annulaire (26) entourant l'espace d'entrée (10) du côté tourné vers le disque de membrane (6), **caractérisée en ce que** le disque de membrane (6) est pourvu radialement à l'intérieur des ouvertures (20) d'une saillie (28, 28a) de forme circulaire d'une seule pièce, la saillie (28, 28a) présentant au moins dans la région opposée au siège de soupape (12) une surface plane (30) tournée vers le siège de soupape (12), qui vient en prise avec le siège de soupape (12) dans une région large, de forme annulaire, **en ce que** des saillies identiques (28, 28a) sont prévues sur le côté supérieur (32) et sur le côté inférieur (34) du disque de membrane (6), et **en ce que** la hauteur de la saillie (28, 28a) est supérieure ou égale à l'épaisseur de paroi du disque de membrane (6) dans la région présentant les ouvertures (20) entre le bourrelet annulaire (14) et la saillie (28).

2. Soupape anti-retour selon la revendication 1, **caractérisée en ce que** la saillie (28, 28a) présente un renfoncement (36) dans une région circulaire centrale.

3. Soupape anti-retour selon la revendication 2, **caractérisée en ce que** l'épaisseur de paroi du fond (38) du renfoncement (36) correspond approximativement à l'épaisseur de paroi du disque de membrane (6) dans la région contenant les ouvertures (20).

4. Soupape anti-retour selon l'une quelconque des revendications précédentes, **caractérisée en ce que** l'arête supérieure circulaire (40) de la saillie (28, 28a) présente un biseau (42) ou un arrondi.

5. Soupape anti-retour selon l'une quelconque des revendications précédentes, **caractérisée en ce que** la paroi latérale annulaire (44) de la saillie (28, 28a) est réalisée de manière inclinée obliquement vers l'extérieur.
